Europäisches Patentamt

European Patent Office

Office européen des brevets

(11) Publication number: **0 140 242**
**B1**

# EUROPEAN PATENT SPECIFICATION

(45) Date of publication of patent specification: **24.05.89**

(21) Application number: **84112208.8**

(22) Date of filing: **11.10.84**

(51) Int. Cl.⁴: **G 01 N 33/68,**
G 01 N 33/577,
G 01 N 33/541,
G 01 N 33/531

(54) Method for assaying basic fetoprotein and reagent therefor.

(30) Priority: **12.10.83 JP 189223/83**

(43) Date of publication of application:
**08.05.85 Bulletin 85/19**

(45) Publication of the grant of the patent:
**24.05.89 Bulletin 89/21**

(84) Designated Contracting States:
**AT BE CH DE FR GB IT LI NL SE**

(56) References cited:
EP-A-0 045 103
US-A-4 376 110

Chemical Abstracts, vol. 98, May 23 - June 6,
1983, p. 316, no. 194359r, Columbus, Ohio (US)

(73) Proprietor: **Nippon Kayaku Kabushiki Kaisha**
**11-2, Fujimi-cho 1 chome Chiyoda-ku**
**Tokyo (JP)**

(72) Inventor: **Watanabe, Keisuke**
**19-13, Kasuga 4 chome Yatabe-machi**
**Tsukuba-gun Ibaragi (JP)**
Inventor: **Shimozuru, Yasunori**
**615-6, Koshirohazama Yatabe-machi**
**Tsukuba-gun Ibaragi (JP)**
Inventor: **Ishii, Masaru**
**7-6, Higashi-Omiya 3 chome**
**Omiya-shi Saitama (JP)**
Inventor: **Taniguchi, Masaru**
**17-12, Konakadai 3-chome**
**Chiba-shi Chiba (JP)**
Inventor: **Hattori, Nobu**
**3-32, Izumigaoka 1 chome**
**Kanazawa-shi Ishikawa (JP)**

(74) Representative: **Eitle, Werner, Dipl.-Ing. et al**
**Hoffmann, Eitle & Partner Patentanwälte**
**Arabellastrasse 4**
**D-8000 München 81 (DE)**

Courier Press, Leamington Spa, England.

## Description

This invention relates to a method for assaying basic fetoprotein and a reagent therefor.

Basic fetoprotein (BFP), which is a basic protein having a molecular weight of 73,000 and an isoelectric point of 9.3 and migrates in the γ-globulin region in electrophoresis, has been found by one of the inventors in serum, intestinum and brain tissues of a human fetus. It has been particularly called basic fetoprotein since it is basic in contrast to the known acidic fetoprotein. Furthermore one of the inventors has established a radioimmunoassay system of said protein and found that assay of said protein in serum would help to diagnose cancer and to judge its condition and therapeutical effect thereon. In particular, it has been found that said fetoprotein is useful to examine the presence of cancer, differing from α-fetoprotein which is useful to diagnose cancer of a particular organ.

These prior findings are described in the following references (1) to (7) which are cited as referential information of the present invention.

(1) Ishii, M. et al.: Studies on feto-neoplastic antigen. Proceedings of the 34th General Meeting of Japan Cancer Society, p. 173 (1975).

(2) Ishii, M.: Studies on novel basic fetoprotein found in various malignant tumors. Igaku no Ayumi, *100* (3), 344—346 (1977).

(3) Ishii, M.: Basic fetoprotein. Igaku no Ayumi, *106* (5), 273—281 (1978).

(4) Ishii, M.: A new carcinoembryonic protein characterized by basic property. Scandinav. J. Immunol., *8* (Suppl. 8), 611—620 (1978).

(5) Ishii, M.: Characterization of basic fetoprotein and clinical usefulness of BFP for immunodiagnosis of human cancer. Carcino-Embryonic Proteins. Chemistry, Biology, Clinical Application, Vol. 1, Lehmann, F.—G. (ed.), Elisevier/North-Holland Biomedical Press, Amsterdam, 333—340 (1979).

(6) Ishii, M., Nishimura, K., Hattori, M., Kanda, Y. and Ishihara, A.: Postoperative surveillance in patients with stomach cancer and monitoring of immuno- and polychemotherapy in patients with leukemia by basic fetoprotein. Carcino-Embryonic Proteins. Chemistry, Biology, Clinical Application, Vol. 2 Lehmann, F.—G. (ed.), Elisevier/North-Holland Biomedical Press, Amsterdam, 603—606 (1979).

(7) Ishii, M.: Clinical usefulness of basic fetoprotein for immunodiagnosis of human cancer. Compendium of Assay for Immunodiagnosis of Human Cancer, Development in Cancer Research, Herberman, R. B. (ed.), Vol. 1, Elisevier/North-Holland Biomedical Press, New York, 45—50 (1979).

Since basic fetoprotein is present in the serum of cancer patients in a trace amount, it is necessary to establish a high sensitive assay system to determine it. RIA and EIA methods have been mainly developed therefor. Detailed descriptions of the RIA method are found in the references (3) to (5) as cited above. Detailed descriptions of the EIA method are found in the following references (8) and (9) which are also cited as referential information.

(8) Ishii, M. et al: Basic fetoprotein, present clinical function test—enforcement and interpretation thereof. Nippon Rinsho *37*, 1536—1539 (1979).

(9) Ishii, M.: Basic fetoprotein: Rinsho Kensa *24* (8), 931—936 (1980).

Subsequently the inventors have prepared monoclonal antibasic fetoprotein antibodies in order to mass-produce the antibodies used in the RIA and EIA methods and to further heighten the sensitivity of these methods by the use of said antibodies. Preparation and evaluation of said monoclonal antibodies are described in the following reference (10).

(10) Proceedings of the 10th Japan Clinical Immunology Assoc. (Osaka). Preparation of monoclonal antibodies for basic fetoprotein.

The reaction specificities for basic fetoprotein of the monoclonal antibodies thus obtained were examined by the EIA and MO methods. Consequently it was found that there are at least three different antigenic determinants on the basic fetoprotein molecule and that the monoclonal antibodies might be classified into three types corresponding to each antigenic determinant. Examples of monoclonal antibodies corresponding to the three antigenic determinants A, B and C are as follows:

| Antigenic determinant | Monoclonal antibody |
|---|---|
| A | 5C4, 5C5, 5C6, 7D1, K1 |
| B | 5B2, 5B3, 5C2 |
| C | 5A-2, 5A3, 5D3, 7D3, 8A2, 7B4, 5D6-2, 8A1, 7A5-2, 8A5, 7B6 |

Under these circumstances, we have studied to establish a highly sensitive method for assaying basic feto-protein by a sandwich method with the use of monoclonal antibodies from various viewpoints. As a result, we have found that monoclonal antibodies which react with antigenic determinants different from each other should be combined as the two antibodies used in the sandwich method. That is to say, combinations of 5C4 and 5C2 or K1 and 5B3 monoclonal antibodies in the foregoing example may be selected. Thus the present invention has been completed to provide a method for assaying basic fetoprotein based on these findings.

Accordingly, is is an object of the present invention to provide a highly sensitive method for assaying basic fetoprotein by a sandwich method. The present invention discloses a technique for selecting monoclonal antibasic fetoprotein antibodies which react with antigenic determinants different from each other as the two antibodies used in the sandwich method to thereby achieve the abovementioned object.

Now the present invention will be described in detail.

As described above, the basic fetoprotein assayed by the method of the present invention is a basic protein which has a molecular weight of 73,000 and an isolectric point of 9.3, migrates in the γ-globulin region in electrophoresis and serves as a tumor marker having a low organ specificity.

Monoclonal antibasic fetoprotein antibodies used in the present invention may be prepared, for example, in the following manner. Antibody-forming cells obtained from a BALB/C mouse sensitized with basic fetoprotein are fused with myeloma cells, P3-X63-Ag8-U1, by a variation of the method reported by Herzenberg et al. Subsequently the antibody titer of the fused cells selected by a HAT medium was assayed by plate binding assay with the use of basic fetoprotein labelled with $^{125}I$. Some cell strains showing high antibody titers are obtained by cloning fused cells which exhibit a high antibody titer and excellent proliferation by limiting dilution. Finally each cell strain thus obtained is transplanted into the abdominal cavity of a mouse and its hydroperitoneum thus obtained is salted out with ammonium sulfate, dialyzed and subjected to DEAE-cellulose column chromatography to collect an IgG component, thereby obtaining some monoclonal antibodies available in the present invention. The monoclonal antibodies thus obtained may be classified into three types corresponding to the three antigenic determinants of basic fetoprotein by the MO and EIA methods as described below.

MO method:

Mixtures of all combinations of two monoclonal antibodies at a ratio of 1:1 are prepared. Formation of a sedimentation line and the presence of fusion between these mixtures and basic fetoprotein are observed by the MO method. When an obvious sedimentation line is observed, the monoclonal antibodies constituting the mixture are classified into different groups which react with antigenic determinants different from each other. On the other hand, when no sedimentation line is observed, they belong to the same group which reacts with an antigenic determinant. When an unobvious sedimentation line makes it impossible to judge definitely, the following EIA method may be employed.

EIA method:

The procedure of Example 1 is followed except that two monoclonal antibodies selected arbitrarily are used instead of the K1 and 5C2 antibodies to prepare reagents of every combination of the monoclonal antibodies. Coloration value ($OD_{405nm}$) is determined by EIA operations. When coloration is observed, the monoclonal antibodies constituting the combination are classified into different groups which react with antigenic determinants different from each other. On the contrary when no coloration is observed, they belong to the same group which reacts with an antigenic determinant.

Thus the monoclonal antibodies can be classified into three groups corresponding to the three different types of antigenic determinants of basic fetoprotein by the MO and EIA methods.

Now the method for assaying of the present invention will be described in detail.

The method for assaying of the present invention is a sandwich method with the use of two antibodies and carried out by taking advantage of either enzyme immunoassay or radioimmunoassay. Usual procedures of these assays are well known per se. Thus the method of assaying of the present invention by enzyme immunoassay may be carried out as follows.

The whole assay system consists of a solid phase, an antibody for coating the solid phase (the first antibody), basic fetoprotein (a standard antigen and test serum), a marker antibody (the second antibody), an enzyme and a substrate. A well of a microtiter-plate for enzyme immunoassay may be used as the solid phase. Prior to the assay, one of the monoclonal antibodies of the present invention is selected arbitrarily as the antibody for coating the solid phase (i.e. the first antibody), dissolved in a 0.1 M phosphate buffer phsiological saline (pH 7.2) to give a protein concentration of $OD_{280nm}$ of 0.050, stored, for example, in a polystyrene well for enzyme immunoassay and allowed to stand overnight at 4°C to thereby coat the surface of the solid phase with the first antibody. The standard antigen may be prepared by purifying hydroperitoneum obtained from a patient suffering from hepatoma to prepare purified basic fetoprotein and diluting it to a certain concentration with a standard antigen diluent.

A preferred marker antibody (i.e. the second antibody) is a monoclonal antibody of the present invention which reacts with an antigenic determinant different from the one with which the first antibody reacts. Example of available enzymes are alkali phosphatase, glucose oxidase, peroxidase and β-galactosidase. Prior to the assay, the marker antibody may be bonded to the enzyme with a bonding agent such as glutaraldehyde to form a conjugate which may be used as a part of the reagent of the present invention. The substrate may be arbitrarily selected depending on the enzyme to be used. For example, p-nitrophenyl phosphate may be used when alkali phosphatase is selected as the enzyme.

The assay may be carried out in a conventional manner of enzyme imminoassay. Thus, as shown in Examples as described below, it may be carried out by introducing a standard antigen or test serum into a cup coated with the first antibody to incubate, subsequently adding an enzyme marker antibody (e.g. the second antibody/alkali phosphatase conjugate) to incubate, finally adding the substrate (e.g. p-nitrophenyl phosphate) to incubate and determining the amount of a decomposed substrate with a spectrophotometer.

The first antibody used for coating the solid phase may be either a single monoclonal antibody or a mixture of several monoclonal antibodies. That is to say, it is not the determining factor in the present invention whether the first and the second antibodies consist of a single or several monoclonal antibodies so long as the first and the second antibodies react with antigenic determinants different from each other.

As shown in Experimental Example as described below, the method for assaying of the present invention is characterized in that similar effects may be obtained in determining the coloration value of the standard antigen and basic fetoprotein value (BFP value) in normal human serum to those obtained by using polyclonal antibodies.

The reagent of the present invention is directly available for said method for assaying of the present invention to achieve the same object as that of the method. Accordingly said reagent may be used in the sandwich method with the use of two antibodies by taking advantage of either enzyme immunoassay or radioimmunoassay. Now an example of an embodiment of the reagent of the present invention used in enzyme immunoassay will be illustrated. The reagent of the present invention is a set which essentially consists of an antibody for coating a solid phase (i.e. the first antibody) and a marker antibody (i.e. the second antibody) optionally with other component(s) selected from a solid phase, a standard antigen, an enzyme and a substrate. When a solid phase is included in the set, said solid phase may be coated with the first antibody. When an enzyme is included in the set, said enzyme may be bonded to the second antibody to thereby form a conjugate. These sets may also be referred to as the reagent of the present invention. Furthermore, other agents suitable for carrying out the assay conveniently, such as an antigen diluent, a reaction diluent, a reaction terminating solution, a substrate solution or a buffer solution, may be included in the set without limiting the present invention.

Brief description of the drawing

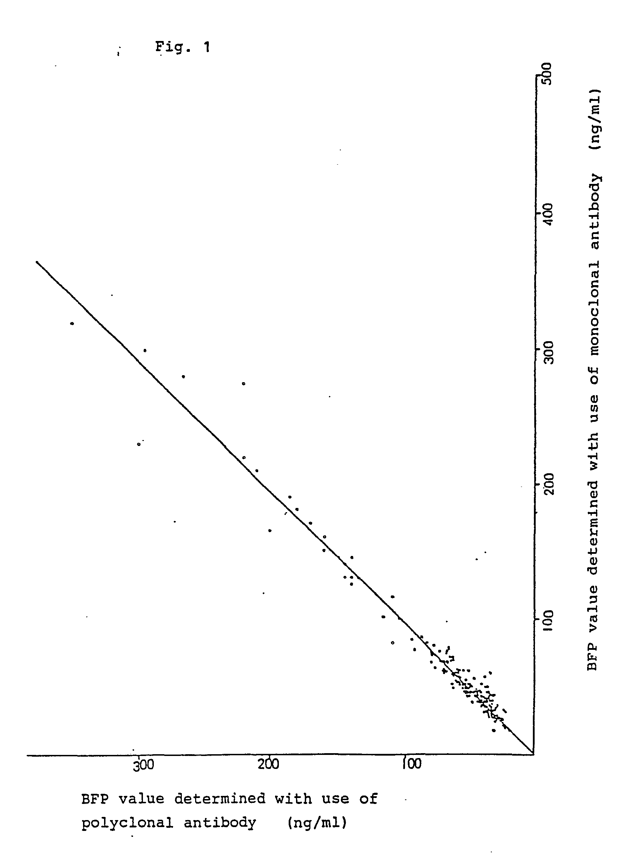

Fig. 1, which corresponds to Fig. 1 described in Experimental Example 1, is a graph showing a correlation between BFP values each determined by assaying with the use of monoclonal antibasic fetoprotein antibodies and by assaying with the use of polyclonal antibasic fetoprotein antibodies.

To illustrate the effect of the present invention, the following Experimental Example will be given.

Experimental Example 1
Sample:
A solid phase first antibody and an enzyme marker antibody were prepared from monoclonal antibasic fetoprotein antibodies in a similar manner as described in Example 1 as shown below.

On the other hand, another solid phase first antibody and an enzyme marker antibody were prepared from polyclonal antibasic fetoprotein antibodies in the following manner.

Serum which was separated from blood obtained from a rabbit immunized with basic fetoprotein thrice was salted out and purified by means of affinity column chromatography using basic fetoprotein to thereby obtain polyclonal antibasic fetoprotein. Subsequently a portion thereof was dissolved in a 0.1 M PB (pH 7.2) to give a protein concentration of $OD_{280nm}$ of 0.05, poured into a well of a microtiter plate for enzyme immunoassay and allowed to stand overnight. The liquid was removed and the residue was washed with demineralized water and dried by a cup drier to thereby obtain a solid phase first antibody. Another portion of polyclonal antibasic fetoprotein was mixed with alkali phosphatase to give a concentration of approximately 1 mg/ml and a 2% glutaraldehyde was added slowly to give a final concentration of 0.2%. After allowing to react for 30 min. at room temperature, the reaction mixture was dialyzed against a 0.05 M Tris hydrochloride buffer solution and 100% NRS was added to give a final concentration of 25%. After adjusting to an optimum concentration with a 0.05 M Tris hydrochloride buffer solution (pH 8.0) which contained 10% of NRS, 1 mM of $MgCl_2$ and 0.9% of NaCl, an enzyme marker antibody was obtained.

Reaction diluents prepared in a similar manner as described in Example 1 were used in a system with the use of monoclonal antibasic fetoprotein antibodies, while reaction diluents prepared in the following manner were used in a system with the use of polyclonal antibasic fetoprotein antibodies. That is to say, normal rabbit serum, trisodium ethylenediaminetetraacetate, and sodium chloride were added to a 0.1 M carbonate buffer solution (pH 9.0) to give concentrations of 12% (20% when a standard antigen was diluted), 10 mM and 0.9%, respectively. A substrate solution and a reaction terminating solution were prepared in the same manners as described in Example 1. 180 examples of normal human serum and 19 examples of that of cancer patients were prepared as specimens.

Method:
BFP values (ng/ml) of all specimens were determined by assaying with the use of monoclonal antibasic fetoprotein antibodies and polyclonal antibasic fetoprotein antibodies respectively in the following manner.

A specimen and a standard antigen having a known concentration were diluted 11-fold with a reaction diluent and poured into wells which has been washed previously in 100 μl portions and incubated for one hour at 37°C. After washing with distilled water, 100 μl of an enzyme marker antibody of the optimum concentration was added and incubated for one hour at 37°C. After rewashing with distilled water, 100 μl of a substrate solution was added and incubated for one hour at 37°C. Subsequently 100 ml of a 1N NaOH was added to stop the reaction and the optical density was determined at 405 nm ($OD_{405nm}$) to calculate the BFP value.

Results:

Fig. 1 shows the result. In Fig. 1, the abscissa refers to the BFP value determined by the assay with the use of monoclonal antibasic fetoprotein antibodies, while the ordinate refers to the BFP value determined by the assay with the use of polyclonal antibasic fetoprotein antibodies. Accordingly, Fig. 1 is a graph showing a correlation between these BFP values. The equation of the regression line in the graph as $y = 1.016x + 0.06$ and the correlation coefficient $\gamma$ is 0.981. Thus these BFP values exhibit an excellent correlation with each other, which suggests that the method of the present invention may bring about a similar result to that obtained by assaying with the use of polyclonal antibodies.

To further illustrate the present invention, the following examples will be given.

Example 1

To 5 ml portions of mouse hydroperitoneum which contained monoclonal antibodies K1 and 5C2 each reacting with antigenic determinants of basic fetoprotein different from each other, a saturated ammonium sulfate solution (4.05 M) was added to give a final concentration of 1.8 M and stirred for two hours at room temperature. Each mixture was centrifuged at 12,000 rpm for 20 min to thereby separate precipitate from supernatant. The precipitate was dissolved in 3 ml of a 0.01 M PBS (pH 8.0) and dialyzed against the buffer solution used to dissolve the precipitate. The dialyzate was poured into a DEAE-cellulose column to elute the IgG component with a 0.1 M PB (pH 8.0). The eluted IgG component was concentrated by a collodion pack (MW 75,000) to prepare K1 and 5C2. The K1 was dissolved in a 0.1 M PB (pH 7.2) to give a protein concentration of $OD_{280nm}$ of 0.05 and the solution was poured into a well of a microtiter plate for enzyme immunoassay and allowed to stand overnight. After removing the liquid, the residue was washed with demineralized water and dried by a cup drier to prepare a solid phase first antibody.

On the other hand, the 5C2 and alkali phosphatase (5000 U/ml) were each dialyzed against a 0.075 M PBS. The IgG component was mixed with alkali phosphatase to give a concentration of approximately 1 mg/ml and a 2% glutaraldehyde was added slowly with a microsyringe to give a final concentration of 0.2%. The mixture was allowed to react at room temperature for 30 min and dialyzed against a 0.05 M Tris hydrochloride buffer solution (pH 8.0). After adding 100% NRS to give a final concentration of 25% and calibrating, the resulting solution was lyophilized to prepare an enzyme marker antibody.

Separately reaction diluents, a substrate solution, a reaction terminating solution and standard antigen diluents were prepared in the following manner.

Normal rabbit serum, normal mouse serum (inactivated at 56°C for 30 min), trisodium ethylenediaminetetraacetate, sodium chloride and sodium triazide were added to a 0.05 M Tris hydrochloride buffer solution (pH 8.0) to give concentrations of 11.8%, 2%, 10 mM, 0.15 M and 0.1%, respectively, to thereby prepare reaction diluents.

p-Nitrophenyl phosphate was added to a 0.05 M carbonate buffer solution (pH 9.4) containing 2 mM of $MgCl_2$ to give a concentration of 4 mg/ml to thereby prepare a substrate solution.

1 N-NaOH was prepared as a reaction terminating solution.

Normal mouse serum (inactivated at 50°C for 30 min), trisodium ethylenediaminetetraacetate, sodium chloride and sodium triazide were added to a 0.05 M Tris hydrochloride buffer solution (pH 8.0) to give concentrations of 18.1%, 10 mM, 0.15 m and 0.1%, respectively, to prepare standard antigen diluents.

Accordingly a set consisting of the foregoing solid phase first antibody, enzyme marker antibody, reaction diluents, substrate solution, reaction terminating solution and standard antigen diluents and lyophilized hydroperitoneum of a hepatoma patient which was employed as a standard antigen was obtained as the reagent of the present invention.

Example 2

Monoclonal antibody 5C2 was dissolved in a 0.05 M phosphate buffer solution (pH 7.5) to give a protein concentration of 1 mg/ml. To 10 µl of the obtained solution, 50 µl of $3.7 \cdot 10^7$ Bq (1 mCi) of $^{125}$I-Na and 10 µl of a solution prepared by dissolving chloramine T in said phosphate buffer solution to give a concentration of 1.5 mg/ml were added successively and the reaction mixture was stirred for 30 sec. Subsequently 100 µl of a solution prepared by dissolving sodium metabisulfite in said phosphate buffer solution to give a concentration of 2 mg/ml was added to stop the reaction. To the reaction liquor, 100 µl of a solution prepared by dissolving potassium iodide in said phosphate buffer solution to give a concentration of 10 mg/ml was added and the reaction mixture was immediately subjected to gel-filtration with Sephadex® G-50 to thereby separate the substance labelled with $^{125}$I from $^{125}$I. Specific radioactivity of the substance labelled with $^{125}$I thus obtained was approximately 18.5 to $74.0 \cdot 10^7$ Bq/µg (5 to 20 µCi/µg).

The second antibody labelled with $^{125}$I thus obtained was combined with the solid phase first antibody prepared in the same manner as described in Example 1 to give the reagent of the present invention for radio-immunoassay.

**Claims for the Contracting States: BE CH DE FR GB IT LI NL SE**

1. A method for assaying basic fetoprotein by the sandwich method using two antibodies, said two antibodies being selected from monoclonal antibasic fetoprotein antibodies which each react with antigenic determinants of the basic fetoprotein different from each other.

2. A reagent for assaying basic fetoprotein by the sandwich method containing two antibodies, said two antibodies being selected from monoclonal antibasic fetoprotein antibodies which each react with antigenic determinants of the basic fetoprotein different from each other.

**Claims for the Contracting State: AT**

1. A method for assaying basic fetoprotein by the sandwich method using two antibodies, said two antibodies being selected from monoclonal antibasic fetoprotein antibodies which each react with antigenic determinants of the basic fetoprotein different from each other.

2. A method for preparing a reagent for assaying basic fetoprotein by the sandwich method containing two antibodies, said two antibodies being selected from monoclonal antibasic fetoprotein antibodies which each react with antigenic determinants of the basic fetoprotein different from each other.

**Patentansprüche für die Vertragsstaaten: BE CH DE FR GB IT LI NL SE**

1. Verfahren zur Bestimmung von basischem Fetoprotein nach der Sandwich-Methode unter Verwendung von zwei Antikörpern, wobei die zwei Antikörper ausgewählt werden aus monoklonalen Anti-basisch Fetoprotein-Antikörpern, die jeweils mit voneinander verschiedenen antigenen Determinanten von basischem Fetoprotein reagieren.

2. Reagens zur Bestimmung von basischem Fetoprotein nach der Sandwich-Methode, welches zwei Antikörper enthält, wobei die beiden Antikörper ausgewählt sind aus monoklonalen Anti-basisch Fetoprotein-Antikörpern, welche jeweils mit voneinander verschiedenen antigenen Determinanten von basischem Fetoprotein reagieren.

**Patentansprüche für den Vertragsstaat: AT**

1. Verfahren zur Bestimmung von basischem Fetoprotein nach der Sandwich-Methode unter Verwendung von zwei Antikörpern, wobei die zwei Antikörper ausgewählt werden aus monoklonalen Anti-basisch Fetoprotein-Antikörpern, die jeweils mit voneinander verschiedenen antigenen Determinanten von basischem Fetoprotein reagieren.

2. Verfahren zur Herstellung eines Reagens zur Bestimmung von basischem Fetoprotein nach der Sandwich-Methode, wobei das Reagens zwei Antikörper enthält, und die beiden Antikörper ausgewählt sind aus monoklonalen Anti-basisch Fetoprotein-Antikörpern, welche jeweils mit voneinander verschiedenen antigenen Determinanten von basischem Fetoprotein reagieren.

**Revendications pour les Etats contractants: BE, CH DE FR GB IT LI NL SE**

1. Procédé d'analyse de la foetoprotéine basique par l'essai en sandwich utilisant deux anticorps, lesdits deux anticorps étant choisis parmi des anticorps monoclonaux antifoetoprotéine basique qui réagissent chacun avec des déterminants antigéniques de la foetoprotéine basique différents les uns des autres.

2. Réactif pour l'analyse de la foetoprotéine basique par l'essai en sandwich contenant deux anticorps, lesdits deux anticorps étant choisis parmi les anticorps monoclonaux antifoetoprotéine basique qui réagissent chacun avec des déterminants antigéniques de la foetobasique différents les uns des autres.

**Revendications pour l'État contractant: AT**

1. Procédé d'analyse de la foetoprotéine basique par l'essai en sandwich utilisant deux anticorps, lesdits deux anticorps étant choisis parmi des anticorps monoclonaux antifoetoprotéine basique qui réagissent chacun avec des déterminants antigéniques de la foetoprotéine basique différents les uns des autres.

2. Procédé de préparation d'un réactif pour l'analyse de la foetoprotéine basique par l'essai en sandwich contenant deux anticorps, ledsdits deux anticorps étant choisis parmi des anticorps monoclonaux antifoetoprotéine basique qui réagissent chacun avec des déterminants antigéniques de la foetoprotéine basique différents les uns des autres.

Fig. 1

BFP value determined with use of monoclonal antibody (ng/ml)

BFP value determined with use of
polyclonal antibody (ng/ml)